# EUROPÄISCHE PATENTANMELDUNG

(11) **EP 1 618 901 A1**
(43) Veröffentlichungstag der Anmeldung: **25.01.2006**
(21) Anmeldenummer: 05105560.6
(22) Anmeldetag: 22.06.2005
(51) Int. Cl.: A61L 17/06

(54) **Biokompatibles und bioabsorbierbares Naht- und Klammermaterial für chirurgische Zwecke**

(30) Priorität: 23.07.2004 DE 102004036399
(71) Anmelder: Biotronik VI Patent AG, 6340 Baar (CH)
(72) Erfinder: Kuttler, Marc, 12205, Berlin (DE); Müller, Heinz Dr., 91052 Erlangen (DE); Wintsch, Daniel, 91054 Erlangen (DE); Heublein, Bernd Prof. Dr., verstorben (DE)
(74) Vertreter: Eisenführ, Speiser & Partner

(57) **Zusammenfassung**

Die vorliegende Erfindung betrifft ein Naht- und Klammermaterial für chirurgische Zwecke, das zumindest in Teilen aus einer biodegradierbaren Magnesiumlegierung mit folgenden Gewichtsanteilen der Legierungskomponenten besteht:
- Seltenerdmetalle 2.0 bis 4.0 Gew.%,
- Yttrium 3.5 bis 4.5 Gew.%,
- Zirkonium 0.3 bis 1.0 Gew.%,
- kein Aluminium oder allenfalls Aluminium <0.01 Gew.%, und
- Rest < 0.5 Gew.%, insbesondere < 0.3 Gew.%,
wobei Magnesium den auf 100 Gew.% verbleibenden Gewichtsanteil an der Legierung einnimmt.

## Beschreibung

Die Erfindung betrifft ein Naht- und Klammermaterial für chirurgische Zwecke, welches äußerst biokompatibel ist, in der Wunde verbleiben kann, da es vom Körper absorbiert wird, und das aufgrund seiner Zusammensetzung den Schutz vor Wundinfektionen verbessert und den Heilungsprozess fördert.

Unter Naht- und Klammermaterial wird ein beliebiges Material verstanden, dass dazu verwendet wird gleiche oder verschiedene Gewebearten bei Menschen oder Tieren zusammenzuhalten, bis diese in ausreichender Form zusammengewachsen sind. Das Zusammenhalten kann dabei durch unterschiedliche Arten und Ausführungsformen wie z.B. durch Fäden, Drähte, Klammern erfolgen. Dabei sollte gesundes Gewebe möglichst nicht in Mitleidenschaft gezogen werden.

Naht- und Klammermaterialien in permanenter, d.h. kein Abbau über die Zeit, als auch bioabsorbierbarer Form sind schon lange bekannt und in Anwendung. Die bisherigen Lösungen haben aber vielfältige ungelöste Nachteile.

Bioabsorbierbares Nahtmaterial in Draht- und Fadenausführungen aus Magnesium ist seit langem bekannt (DE 630061, DE 676059, DE 665836, DE 688616), hat sich aber in der Vergangenheit aufgrund von technologischen Schwierigkeiten nicht etabliert. Hier sind insbesondere eine starke Gasentwicklung, ein ungleichmäßiges Degradationsverhalten und unzureichende Knickeigenschaften für Knotenbildungen zu nennen.

Früher wurden insbesondere Katgut und Collagenmaterialien als bioabsorbierbares Nahtmaterial in Fadenausführung verwendet. Heutzutage finden insbesondere synthetisch gewonnene Polymere Anwendung (US 3,565,869). Nachteil der Polymere aber ist u.a. eine eingeschränkte Biokompatibilität. Die Degradation erfolgt durch einen vom Körper initiierten Abbauprozess, der u.a. mit Entzündungserscheinungen einhergeht. Solches Nahtmaterial ist oft auch aufwändig in der Herstellung und Sterilisation.

Um das Verbinden von Gewebe bzw. das Verschließen von Wunden effizienter zu gestalten, werden inzwischen auch chirurgische Klammern bzw. Tacker aus permanenten Materialien eingesetzt (EP 0 076 744, EP 0 284 345, US 3,643,852). Neben der Zeitersparnis bei der Anwendung, einfacherer Sterilisation und der einfachen Anwendung, gewährleistet das Tackern auch eine bessere Adaptierung und Evertierung der Wundränder, was zu einer besseren Verheilung und deutlich unauffälligeren Narben führt. Nachteilig ist jedoch, dass die Klammerschenkel meist traumatische Kanäle in das Gewebe reißen, was häufig zu Entzündungen führt. Bei der späteren Entfernung der Klammern ist die Gewebezerstörung und damit Entzündungsgefahr noch ausgeprägter. Dies führt, im unkritischsten Fall zu auffälligen Narben die von den Klammerschenkeln herrühren.

Um solche Effekte zu reduzieren, wurden Form und Verformung der Klammern optimiert (DE 26 25 991, DE 32 04 532, US 1,910,688, US 4,321,002, DE 40 14 653). Gewebezerstörungen und Entzündungen bei der Entfernung der Klammern lassen sich aber weiterhin nicht gänzlich vermeiden.

Um die Klammertechnik auch für tiefer liegende Gewebe einsetzen zu können, die nach der Verheilung nicht mehr zugänglich sind, sowie generell Gewebezerstörungen und Entzündungen durch Klammer-Entfernungen zu vermeiden, sowie die nachfolgende Wundversorgung noch effizienter zu gestalten, werden derzeit biodegradierbare Klammern bzw. Tacker entwickelt und getestet. Diese basieren, wie auch das fadenartige Nahtmaterial, auf Polymeren. Damit verbundene Schwierigkeiten sind die mechanische Festigkeit und Verformbarkeit bei geeigneter Baugröße oder das Degradationsverhalten. Eine große Herausforderung sind auch die Entzündungserscheinungen und Infektionen die mit solchen Materialien einhergehen (Chegini et al., J. Report Med. 1988 Feb; 33(2):187-92; Chegini et al., J. Report Med. 1988 Jan; 33(1):25-9).

Im Rahmen der Weiterentwicklung von biodegradierbaren Metallen, mit denen man viele der Nachteile der Polymerlösungen vermeiden kann, werden Magnesiumlegierungen erwähnt, die u.a. auch für Fäden, Drähte oder Klammern verwendbar sein sollen (EP 1 395 297). Die dort vorgeschlagenen Legierungen enthalten Anteile von Seltenerdmetallen und von Lithium. Die Legierung kann Yttrium in einem Anteil von 0.01 bis 7 Gew.% und Aluminium in einen Anteil von 0.01 bis 16 Gew.% enthalten.

Der vorliegenden Erfindung liegt die Aufgabe zugrunde, biodegradierbares Naht- und Klammermaterial für chirurgische Zwecke zu verbessern. Insbesondere soll die Biokompatibilität, verbessert sowie körpereigene Heilungs- und Abwehrmechanismen unterstützt werden.

Diese Aufgabe wird durch das erfindungsgemäße Naht- und Klammermaterial für chirurgische Zwecke mit den im Anspruch 1 genannten Merkmalen gelöst. Das Naht- und Klammermaterial besteht zumindest in Teilen aus einer biodegradierbaren Magnesiumlegierung mit folgenden Gewichtsanteilen der Legierungskomponenten:
- Seltenerdmetalle 2.0 bis 4.0 Gew.%,
- Yttrium 3.5 bis 4.5 Gew.%,
- Zirkonium 0.3 bis 1.0 Gew.% und
- Rest < 0.5 Gew.%, insbesondere < 0.3 Gew.%,

wobei Magnesium den auf 100 Gew.% verbleibenden Gewichtsanteil an der Legierung einnimmt. Die genannten Magnesiumlegierungen zeichnen sich gegenüber den bisher bekannt gewordenen Magnesiumlegierungen dadurch aus, dass für das Einsatzgebiet ausreichende mechanische Eigenschaften besitzen sowie ein kontrolliertes Abbauverhalten zeigen, das Gasausscheidungen beim Abbau auf ein sehr niedriges und für die gewünschte Anwendung tolerierbares Niveau drückt.

Ferner wurde im Rahmen der Verwendung von solchen Magnesium-Legierungen in In-Vivo und In-Vitro Versuchen gezeigt, dass diese Legierungen und Ihre Abbauprodukte äußerst biokompatibel sind. Überraschender Weise hat sich dabei herausgestellt, dass die Verwendung solcher Magnesium-Legierungen starken immunologischen oder Entzündungsreaktionen des Körpers entgegenwirkt. Auch eine kontrolliertes Zellwachstum, insbesondere glatter Muskelzellen und Endothel-Zellen, konnte in In-Vitro Versuchen belegt werden. Wucherungen, die die Quelle starker Vernarbungen sind, scheinen verhindert bzw. stark eingedämmt zu werden. Der den positiven Effekten zugrunde liegende Wirkungsmechanismus ist bisher im Detail nicht ergründet.

Allgemein bekannte Wirkungen und Einflüsse des üblicherweise über die Ernährung aufgenommenen Magnesiums auf die Körperfunktionen lassen vermuten, dass solche Prozesse auch durch die direkten Verwendung von Magnesium bzw. der Magnesium-Legierungen und die bei der Degradation aufgenommenen Zerfallsprodukte zumindest lokal aktiviert werden.

Es ist z.B. bekannt, dass Magnesium im Organismus einen positiven Einfluss auf die Wundheilung hat, da dieses für den anaeroben Stoffwechsel notwendig ist und die normale Granulation des Bindegewebes, d.h. auch eine schnelle Heilung der u.a. durch die Naht verursachten Gewebewebeschäden, fördert (Dr. med. Dr. sc. Nat PG Seeger, SANUM-Post Nr. 13/1990, 14-16). Auch im Hinblick auf antimikrobielle Wirkungen vom Magnesium weiß man, dass die unspezifische Abwehr über das Properdinsystem nur bei Anwesenheit von Magnesium wirksam ist und die Phagozytose von Bakterien durch Leukozyten eine Anregung durch das Magnesium erfährt. Somit sorgt Magnesium u.a. für die Bekämpfung von Infektionen durch Unterstützung bzw. Aktivierung des körpereigenen Immunsystems und mindert auch generell die Anfälligkeit für Infektionen. Dies hilft u.a. Infektionen im Nahtbereich entgegenzuwirken.

Unter der Sammelbezeichnung "Seltenerdmetall" werden hier die Elemente Scandium (Ordnungszahl 21), Lanthan (57) sowie die 14 auf das Lanthan folgenden Elemente Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), die als Lanthanoide verstanden.

"Biodegradation" im erfindungsgemäßen Sinne betrifft hydrolytische, enzymatische und andere stoffwechselbedingte Abbauprozesse im lebendem Organismus, die zu einer allmählichen Auflösung zumindest großer Teile der verwendeten Materialien führen. Synonym wird häufig der Begriff Biokorrosion verwendet. Der Begriff Bioresorption umfasst zusätzlich die anschließende Resorption der Abbauprodukte.

Vorzugsweise enthält der Rest der Magnesium kein Aluminium oder der Aluminium-Anteil ist allenfalls <0.01 Gew.%. Es hat sich in Untersuchungen von aluminiumhaltigen Magnesiumlegierungen gezeigt, dass ein Verzicht auf Aluminium oder ein Anteil unterhalb der genannten Grenze an Aluminium die Biokompatibilität der Legierung noch deutlich verbessert. So scheint Aluminium eine toxische Wirkung zu besitzen, die für die hier beschriebenen Anwendungen störend ist.

Weiterhin ist bevorzugt, dass die Magnesiumlegierung einen Neodym-Anteil von 1.5 bis 3.0 Gew.% enthält. Die Gegenwart von Neodym in der Legierung scheint die Materialeigenschaften, vor allem die Verarbeitbarkeit zu Drähten etc. wesentlich zu verbessern.

Ferner ist bevorzugt, dass der Rest die Elemente Lithium und/oder Zink beinhaltet. Diese Elemente beeinflussen offenbar - sofern in geringen Anteilen vorhanden - die Materialeigenschaften in einem positiven Sinne, d.h. Erleichtern die Verarbeitbarkeit und Tragen zum günstigen Degradationsverhalten der Magnesiumlegierung bei. Bevorzugt beträgt ein Lithium-Anteil der Legierung 0.15 - 0.2 Gew.% und ein Zink-Anteil der Legierung 0.004 - 0.2 Gew.%.

Nach einer bevorzugten Variante wird die spezifische Zusammensetzung der Magnesiumlegierung sowie seine Modifikation dahingehend vorgegeben, dass die Zersetzung unmittelbar nach der Verwendung als Naht- bzw. Klammermaterial einsetzt und die mechanische Integrität so lange Aufrecht erhalten bleibt, bis die Halte- bzw. Verschlussfunktion zumindest in Teilbereichen durch das Zusammenwachsen des betroffenen Gewebes nicht mehr erforderlich ist. Dieser Zeitraum sollte vorzugsweise zwischen 1 bis 30 Tage, insbesondere 3 bis 14 Tage, betragen. Der Umfang der Abbauprozesse ist von den am Anwendungsort herrschenden Bedingungen abhängig.

Der Degradationsablauf lässt sich u.a. durch die Materialstärke, Temperaturbehandlungen, Oberflächenbehandlungen, die genaue Zusammensetzung der Legierung oder durch bioabsorbierbare Oberflächenbeschichtungen steuern.

Bevorzugt liegt das magnesiumhaltige Naht- und Klammermaterial als Faden, aus Einzelfäden geflochtener Faden, Draht oder Klammer unterschiedlicher Dicke, Querschnitte und Längen vor. Auch andere Ausführungsformen für die beschriebene Funktionalität sind denkbar.

Das Material lässt sich insbesondere mittels Gamma- und Beta-Bestrahlung oder auch mit alkoholischen Lösungen einfach sterilisieren.

## Patentansprüche

1. Naht- und Klammermaterial für chirurgische Zwecke, das zumindest in Teilen aus einer biodegradierbaren Magnesiumlegierung mit folgenden Gewichtsanteilen der Legierungskomponenten besteht:
- Seltenerdmetalle 2.0 bis 4.0 Gew.%,
- Yttrium 3.5 bis 4.5 Gew.%,
- Zirkonium 0.3 bis 1.0 Gew.% und
- Rest < 0.5 Gew.%, insbesondere < 0.3 Gew.%,
wobei Magnesium den auf 100 Gew.% verbleibenden Gewichtsanteil an der Legierung einnimmt.

2. Naht- und Klammermaterial nach Anspruch 1, **dadurch gekennzeichnet, dass** kein Aluminium enthalten ist oder ein Aluminium-Anteil an der Legierung <0.01 Gew.% ist.

3. Naht- und Klammermaterial nach Anspruch 1, **dadurch gekennzeichnet, dass** die Magnesiumlegierung einen Neodym-Anteil von 1.5 bis 3.0 Gew.% enthält.

4. Naht- und Klammermaterial nach Anspruch 1, **dadurch gekennzeichnet, dass** der Rest Lithium enthält und ein Lithium-Anteil an der Legierung 0.15 - 0.2 Gew.% beträgt.

5. Naht- und Klammermaterial nach Anspruch 1, **dadurch gekennzeichnet, dass** der Rest Zink enthält und ein Zink-Anteil der Legierung 0.004 - 0.2 Gew.% beträgt.

6. Naht- und Klammermaterial nach einem der vorhergehenden Ansprüche, **dadurch gekennzeichnet, dass** ein Degradationsverhalten aufgrund der spezifischen Zusammensetzung der Magnesiumlegierung sowie weiterer Modifikation und Behandlungen dahingehend vorgegeben ist, dass die mechanische Integrität mindestens 1 bis 30 Tage aufrecht erhalten bleibt.
